Europäisches Patentamt

European Patent Office

Office européen des brevets

(19)

(11) Publication number: **0 336 030**

**A1**

(12) # EUROPEAN PATENT APPLICATION

(21) Application number: 88303082.7

(51) Int. Cl.⁴: **A61B 5/10**

(22) Date of filing: 06.04.88

(43) Date of publication of application:
11.10.89 Bulletin 89/41

(84) Designated Contracting States:
AT BE CH DE ES FR GB GR IT LI LU NL SE

(71) Applicant: PHYSICAL HEALTH DEVICES, INC.
Suite 1 Building H 1301 West Copans Road
Pampano Florida 33064(US)

(72) Inventor: Church, John Y.
17345 N.W. 62 Court
Miami Florida 33015(US)
Inventor: Hassel, William R.
1701 S.W. 139 Avenue
Davie Florida 33325(US)
Inventor: Naghdi, Fred
900 S.W. 9th Street Circle No. 101
Boca Raton Florida 33432(US)

(74) Representative: Knott, Stephen Gilbert et al
MATHISEN, MACARA & CO. The Coach
House 6-8 Swakeleys Road
Ickenham Uxbridge Middlesex UB10 8BZ(GB)

(54) A lifting, monitoring and exercise training system.

(57) The invention is directed to a microprocessor based system utilizing electromyographic sensor (22) to monitor muscle force for lift training and exercise training. In the lift training embodiment the electrodes (12, 14, 16) of the electromyographic sensor are secured to a belt (10) so that they are lcoated adjacent to the lower back of the user when wearing the belt. The lift training embodiment is also provided with a goniometer (20) to measure lifting angle during training, which together with muscle force generated by the lower back is compared by the microprocessor to preprogrammed lifting parameters and if these parameters are exceeded the user is warned by an audible indicator (27). The lift trainer embodiment also periodically measures interelectrode impedance to insure actual usage. The exercise training embodiment has a bar graph display (50) displaying muscle intensity and two light emitting diodes (52) alerting a user when to contract or relax a monitored muscle group. The microprocessor is preprogrammed with a exercise routine and alerts a user when the routine is to begin, the duration of muscle contraction and relaxation, and the repetitions required. During exercise the user can monitor muscle intensity from the bar graph display (50) and or auditory feedback element (27). An alternative lift training system comprises a belt mounted goniometer (20) which is operatively coupled to a microprocessor having an electronic memory (25) for time logging a lifting session. To insure actual usage the belt is provided with temperature and/or motion sensors.

Fig.4.

## A LIFTING MONITORING AND EXERCISE TRAINING SYSTEM

The invention is directed to systems for monitoring the lifting motion and/or the exercise training of an individual.

Annually millions of workers suffer from work related low back pain, most of which is attributed to improper lifting techniques. Such injuries result in work time lost and disability claims costing employers large amounts of money each year.

A number of devices have been proposed to monitor and provide feedback as to a person's correct posture. Such devices may comprise longitudinal belts that are wrapped from a person's waist over his or her shoulder, these devices monitor belt tension insuring that the user's back is being held upright, see US-A-3608541 US-A-4007733 and US-A-4055168. Other devices include conventional belts that are fitted with sensors for monitoring stomach sag, which indicates improper posture because of relaxation of the stomach muscles, see US-A-3582935 and US-A-3670320. US-A-3644919, discloses a signaling device indicating the improper position of a skier's legs during skiing.

In addition to monitoring lifting technique and motion it is also important to monitor a person's exercise program during physical therapy to insure that the physical therapy is being done properly, for the correct intensity and duration. Devices for measuring overall physical loads have been proposed, see US-A-4394865; but these devices do not tend to be directed to a specific muscle group for measuring the muscle force used in an exercise or the duration of that exercise.

The amount of force exerted by a muscle is directly related to its enervation by virtue of the amplitude and frequency of constituent action potentials. Therefore it is possible to measure muscle force with electromyographic (EMG) techniques. In integrated electromyography (IEMG) the myoelectric signal is rectified and time averaged to produce an accurate representation of the EMG signal energy which can be related to muscle force.

According to a first aspect of the invention, there is provided a physical movement monitoring system for monitoring the lifting movement of a user characterised in that said system comprises a belt means adapted and constructed to be secured about a user's waist, at least one electromyographic sensor which produces a rectified and time averaged signal forming a muscle force signal, the sensor being provided with at least one electrode which is secured to the belt in such a manner that when the belt is worn the electrode is located adjacent to a user's lower back so that the sensor senses muscles force exerted by the lower back during a lifting operation.

According to a second aspect of the invention there is provided an exercise training system for alerting a user when an exercise is to begin and recording the intensity of the exercise, characterised in that the training system comprises an electromyographic sensor which produces a rectified and time averaged signal forming a muscle force signal, the sensor being provided with at least one electrode that is positioned adjacent to a user's muscle group for indicating muscle force of that muscle group, and a control means having a clock for measuring time intervals and an alerting means for alerting a user that an exercise period has started as determined by the clock for a predetermined time interval loaded into the control means, whereby a user in response to the alterting means contracts and relaxes a predetermined muscle group and the muscle force used in the exercise is sensed by the electromyographic sensor.

According to a third aspect of the invention, there is provided a physical movement monitoring system for monitoring the lifting movement of a user characterised in that said system comprises a belt means adapted and constructed to be secured about a user's waist, a goniometer for measuring horizontal angles being mounted on the belt for measuring the lifting angle of a user's back, and generating an output signal related thereto.

The following is a more detailed description of some embodiments of the invention, by way of example, reference being made to the accompanying drawings in which:-

Figures 1 and 2 are perspective views of a lift training belt secured to a user,

Figure 3 is a top view of the belt of Figures 1 and 2,

Figure 4 is an electrical block diagram of the lift training system of Figures 1 to 3,

Figures 5a-5d are graphs of muscles force and lifting angle versus time for various lifting scenarios using the lift training system of Figures 1 to 4,

Figures 6 is a block diagram of the operating system for the lift, training system of Figures 1 to 5a-d.

Figure 7 is a front view of an exercise training device,

Figure 8 is an electrical block diagram of the exercise training system of Figure 7,

Figure 9 is a flow chart of an auto ranging technique for a bar graph display of the exercise training system of Figures 7 and 8,

Figure 10 is a cross sectional view of a casting using exercise training electrodes of the exercise training system of Figures 7 and 8,

Figure 11 is a side view of a cylindrical mounting assembly for sensing electrodes of an exercise training system that is adapted to be inserted into a female's vagina,

Figure 12 is a side view of cylindrical mounting assembly for a sensing electrode of an exercise training systenm that is adapted to be inserted into a user's anus, and

Figure 13 is an electrical block diagram of an alternate embodiment of a lift training system of the kind shown in Figures 1 to 6.

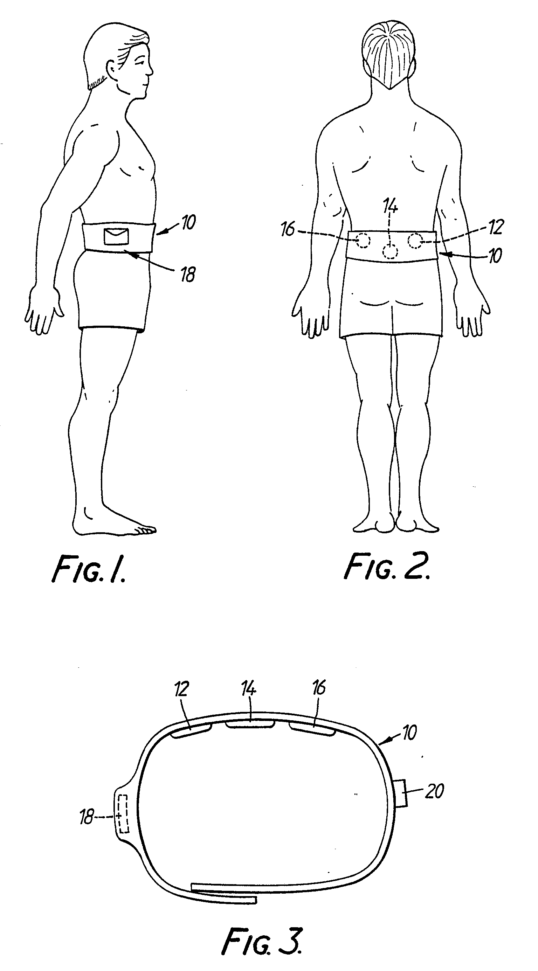

Figures 1 to 3 illustrate the belt mouinted lift training system. Belt 10 is secured to just above the waist of a user in a conventional manner. The belt is provided with three electrodes 12, 14 and 16 which are electrically coupled to monitoring device 18 through wires (not shown in these figures). The elctrodes are secured to the belt so that as the belt is worn the elctrodes are located adjacent to a patient's lower back. The training and monitoring device is located in a pocket on the belt. Goniometer 20 is also mounted o the belt and is located so that it is positioned adjacent to a patient's side so that as a patient bends the goniometer can monitor the bending angle. It should be noted that by mounting the training and monitoring device so that it too is located on the patient's side, the goniometer can be located in the device rather than having a separate mounting location on the belt.

The belt can be fabricated from a light weight elastomeric fabric and is designed to be worn just above the waist. The belt fastener or securing member can be made from hook and pile fasteners located at the adjoining ends of the belt. The electrodes themselves are silver element pads that serve as surface electrodes of an electromyographic sensor. The goniometer and the electrodes are connected to the monitoring device via wires located in the fabric that terminate in metallic snaps that can be coupled to mating snaps located in the training and monitoring device.

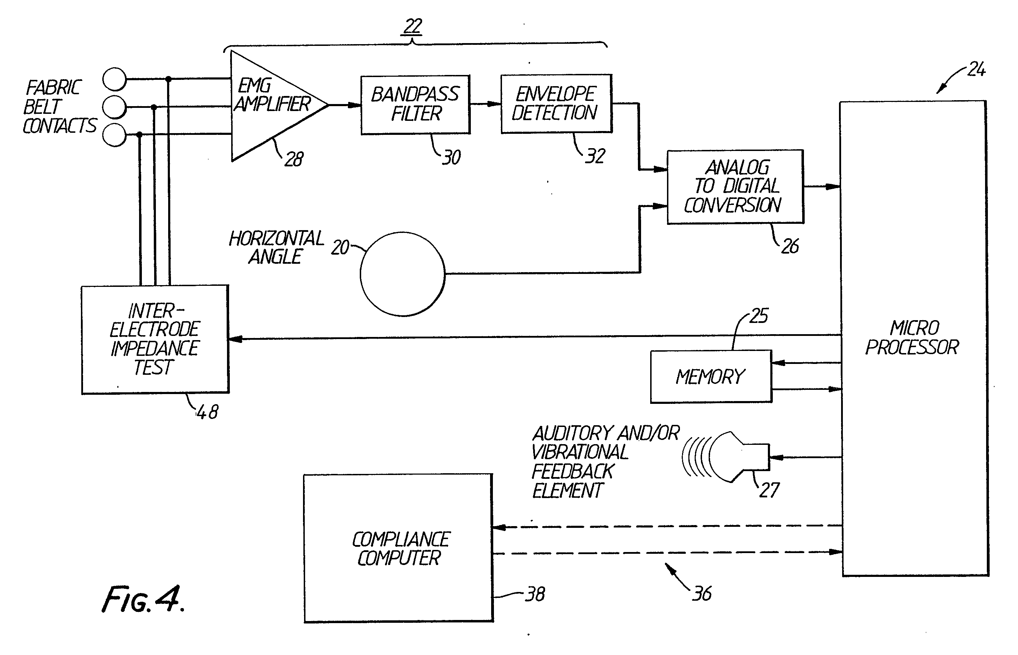

Figure 4 is an electric block diagram of the training and monitoring device. The monitoring device comprises electromyographic sensor 22 which is operatively connected to control means 24 through an analog to digital converter 26. Goniometer 20 is also coupled to the control means through converter 26. The control means comprises a microprocessor unit acting also as an internal clock and is interfaced to an electronic memory 25 that forms a recording means. The microprocessor is coupled to a indicator means 27, which can be auditory and/or vibrational for indicating to the user a lifting condition which exceeds preset parameters programmed into the microprocessor.

In operation the myoelectric signals from the three electrodes are amplified by high gain differential amplifier 28, filtered by bandpass filter 30 and directed to envelope detector 32 which converts the raw EMG waveform of the myoelectric signals into an approximation of the total myoelectric energy which essentially comprises a muscle force signal. As the resulting muscle force signal is an analog signal it is converted into a digital format acceptable to the microprocessor. Similarly the goniometer forms a horizontal angle signal that comprises a lifting angle signal that is also converted from an analog to a digital format before being directed to the microprocessor. It should be noted that goniometer measures the lumbar angle including anterior and/or left/right lateral angles.

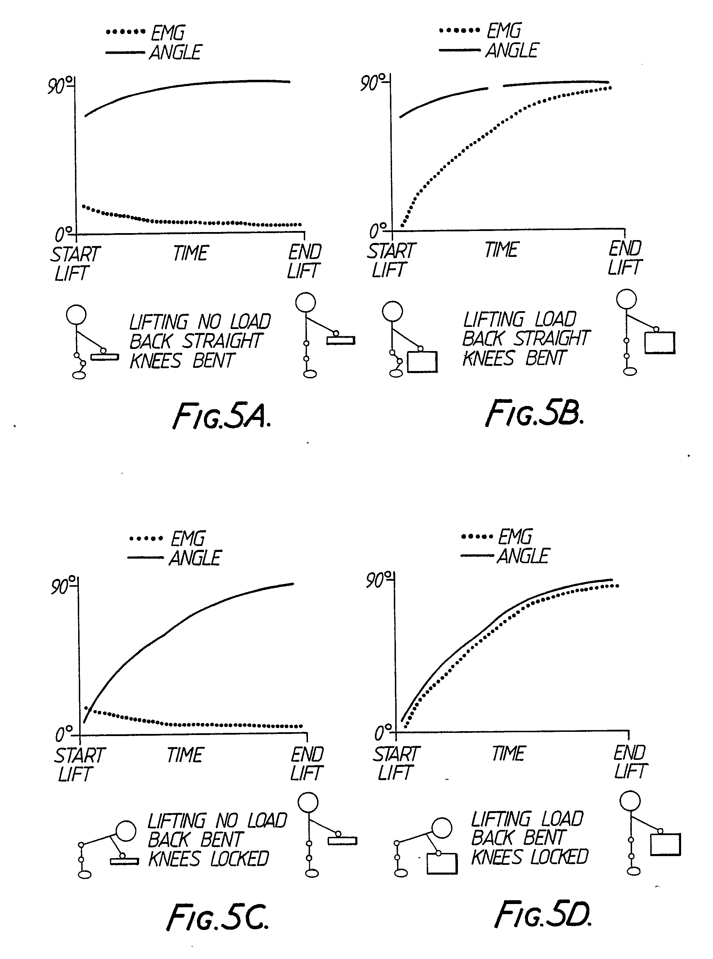

Figure 5 reflects the idealized behaviour of lumbar angle and EMG measurement under several lifting conditions. The EMG curves shown do not include components of inertia and body weight.

Figure 5a and 5c are graphical presentations of lifting no loads in a back straight position and back bent position. As can be seen in the back straight position the horizontal angle changes only slightly whereas in the back bent position the horizontal angle changes from nearly zero degrees to ninety degrees. However since no additional load is involved in either lifting sequence the amount of muscle force (EMG) required is minimal. In Figures 5b and 5d a load is lifted and although the lifting angle is identical to the no load sequence, the amount of muscle force required in each sequence varies considerably because of the lifting methodology. In the back bent position the amount of muscle force required from the lower back tends to mirror the change in lifting angle whereas in the back straight position during the initial lifting motion the amount of lower back muscle force is considerably reduced because the legs are doing the lifting.

In training a user of the system, a teacher programs the microprocessor via the compliance computer 38 with a set of lifting parameters which include limits as to muscle load and horizontal angle. As there is interplay between these parameters the teacher can set up a system wherein a combination of the parameters triggers a feedback warning signal. For example in Figure 5c the user has taken an incorrect lifting angle but since the user is not lifting any load the indicator is not triggered. However in Figure 5d the user has taken an incorrect lifting position and is lifting a load, therefore the indicator is triggered. As such the present system gives the teacher the ability to program triggering parameters that are a combination of the lifting angle and muscle force required.

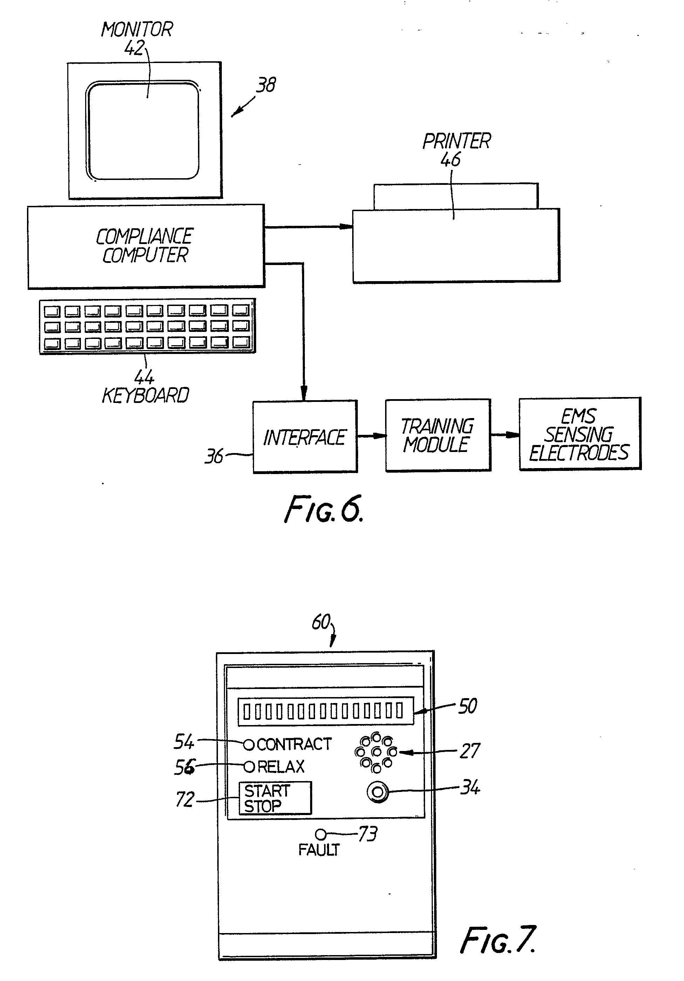

The monitoring system is battery operated and located in a lockable housing so that after the teacher has programmed the microprocessor, the housing is locked and the battery cannot be tampered with by the user. The microprocessor is provided with interface 36 comprising a plug for coupling the microprocessor to compliance computer 38. The compliance computer can be an IBM PC compatible unit and is used to interrogate the memory so that a training session can be tabulated for evaluation by the teacher. In addition this interface can be used for programming the microprocessor with the programmed lifting parameters. As can be seen in Figure 6, the compliance computer is provided with monitor 42, input keyboard 44, and printed 46.

To insure that the monitoring system is operating correctly the microprocessor periodically activates interelectrode impedance test 48 to check if electrode contact is sufficient. The test aplied a bipolar sinusoidal signal across the EMG inputs, the impedance is then measured by the microprocessor. In addition the microprocessor can be provided with a testing system for testing battery voltage to insure proper voltage to the monitoring system. In the event that the contacts fail the impedance test or the battery has insufficient voltage the microprocessor signals the user through the indicator means and turns off the system.

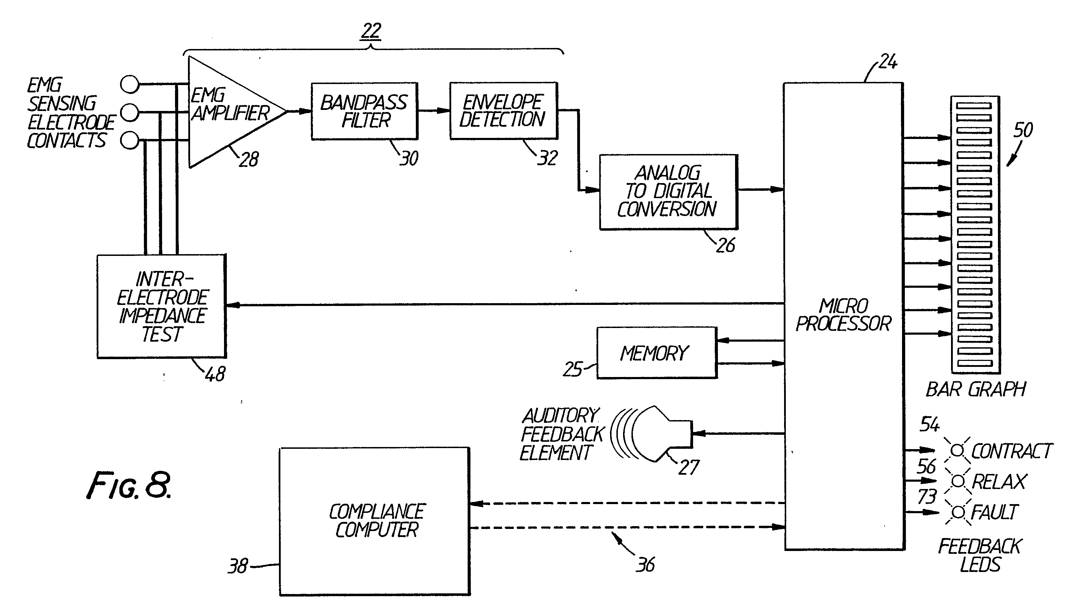

. Figures 7-12 are directed to an exercise training system which is similar to the lift training monitoring system. As can be seen in Figure 8 the circuitry is similar except that the exercise training system is provided with visual feedback display means 50 comprising a bar graph, and alerting means 52 comprising three light emitting diodes.

The auditory feedback element 27, which in the lifting training system is an indicating means, in this embodiment is used in conjunction with the visible display means and the alerting means to inform the patient audibly that these displays have been triggered.

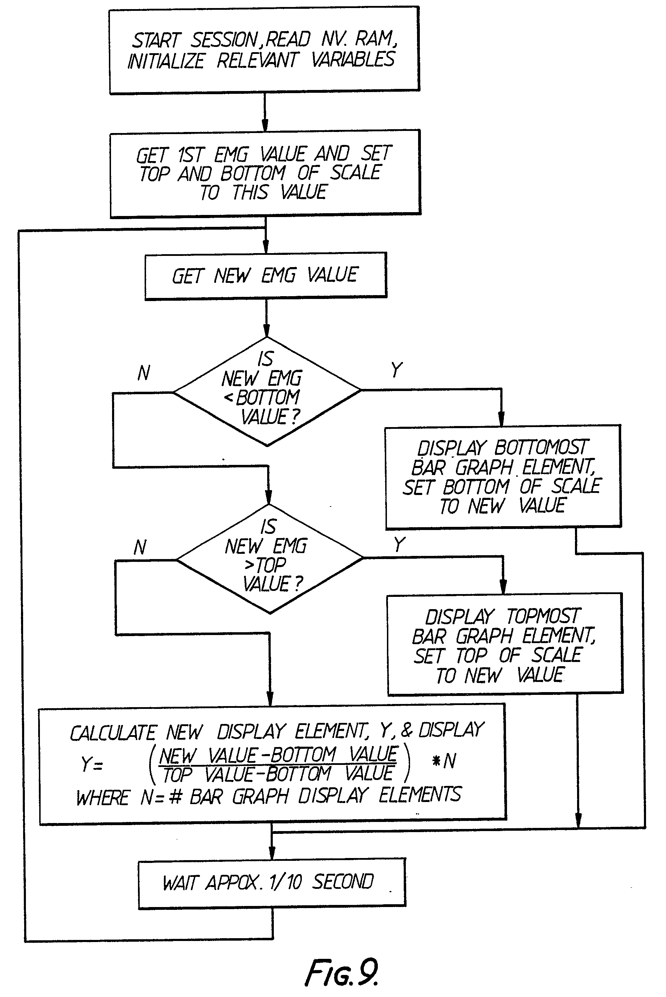

Bar graph 50 is a liquid crystal or light emitting diode display that is used for displaying muscle force used during use. The exercise training system is auto ranging with respect to the bar graph, the alogorithm for auto ranging the bar graph is disclosed in Figure 9. During an exercise period light emitting diode 54 lights up indicating to the user to contract the muscle group that is equipped with the electromyographic electrodes. The user keeps that muscle contracted until light emitting diode 54 is turned off, and light emitting diode 56 lights up indicating to the user to relax the muscle group. Contract/relax cycles are repeated as determined by the preprogrammed microprocessor. The intensity of the muscle contractions is fed back to the user by viewing bar graph 50 which indicates muscle force used.

A physical therapist first applies the electromyographic electrodes to a patient adjacent to the muscle group to be exercised. Then the therapist programs the microprocessor via the compliance computer of the training system, by programming a time interval in which the exercise routine is to begin, the timed interval for contracting a muscle group and relaxing a muscle group, and the number of repetitions. The therapist then couples the unit to the electrode leads and the patient can then conduct his own physical therapy by using isometric exercises for contracting the desired muscle group for the required duration and repetitions and monitoring the intensity of the exercise on the bar graph.

As with the lift training and monitoring system the exercise training system can be coupled to compliance computer 38 through interface 36, whuich can comprise a simple jack. The compliance computer is used to program the microprocessor and to tabulate the patient's performance with the exercise program by interrogating the electronic memory which recorded the exercise session. The therapist can then program into the microprocessor a new training routine based upon the patient's actual performance in the last training session. As with the lift training system the compliance computer is also used to program the microprocessor.

Figure 7 is a front view of the training and monitoring device which is relatively compact. The circuitry including the microprocessor, the electronic memory, and the elctromyographic processing circuitry are contained in housing 60. The device is provided with a start/stop switch 72 for overriding the exercise routine programmed into the microprocessor, and a third light emitting diode 73 indicates the device is not functioning correctly based upon its self testing, which is identical to the self testing of the lift training device.

Figure 9 discloses a flow chart illustrating the method of auto ranging the bar graph display. At the start of an exercise session the exercise parameters programmed into electronic memory via the compliance computer are read by the microprocessor and are used to initialize relevant variables. The auto ranging method then through subsequent EMG (muscle force) readings sets a continually updated top value and bottom value for the bar graph scale. The method then calculates a new EMG reading located between the top and bottom value as a ratio of the EMC range and as such displays this ratio by lighting up the correct number of bar graph display elements.

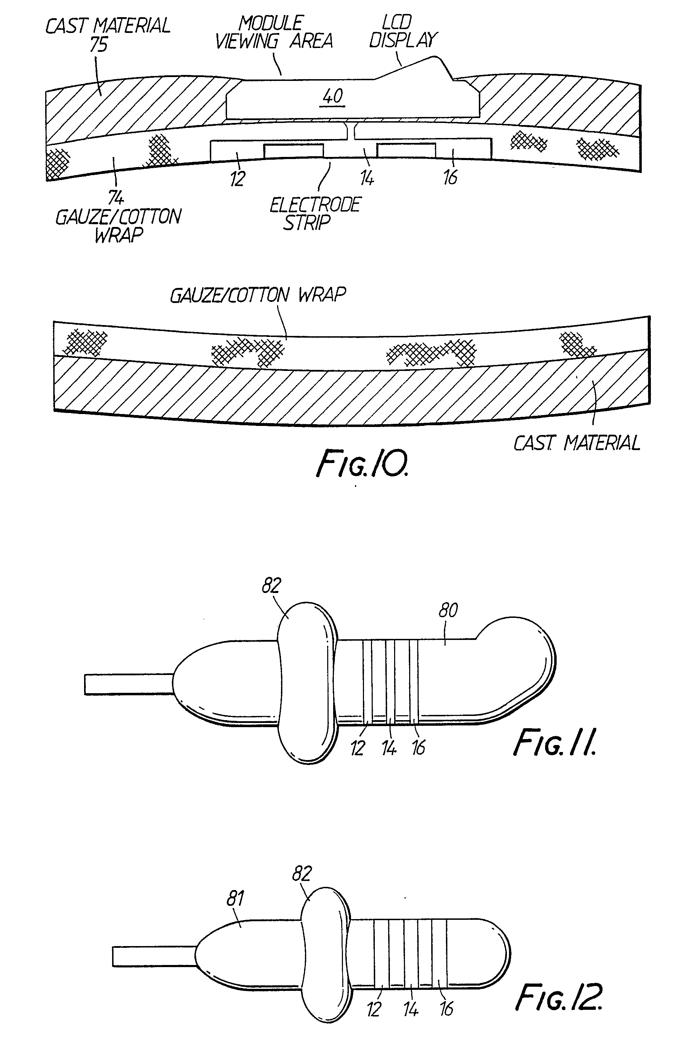

Figure 10-12 disclose different devices for securing the electrodes of the electromyographic sensor to selected body location. In the embodiment illustrated in Figure 10, the electrodes are secured to cotton gauze 74 that forms the inner liner of a cast for a limb. The monitoring housing and related circuitry because of its compact nature, can then be embedded in casting material 75 of the outer cast layer. The bar graph display is located at an angle to the housing to facilitate viewing by the patient.

Figures 11 and 12 are directed to electrode mounting assemblies that were designed to be inserted into a naturally occurring body orifices. These assemblies are cylindrical and have three stainless steel electrode bands located about their circumference.

The embodiment illustrated in Figure 11, comprises cylindrical member 80 which is inserted into a female vagina so that the female patient can monitor the exercise of associated vaginal muscles. The embodiment illustrated in Figure 12 comprises cylindrical member 81 and is inserted in a patient's anus for monitoring a patient's exercise of the anal sphincter muscles. Both units are made from injected moulded plastic, and are provided with depth guages 82 which can be adjustably positioned and fixed on the cylindrical members by the therapist.

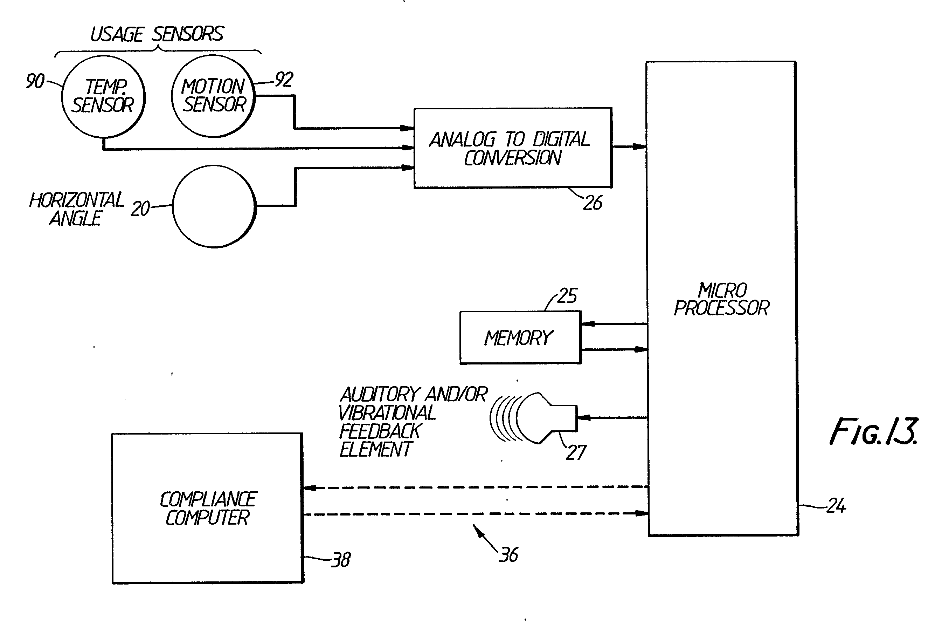

An alternate embodiment of the lift training system is illustrated in Figure 13 and comprises a lift training system that is not provided with an electromyographic sensor. Instead only goniometer 20 is used to measure lumbar angle. Temperature sensor 90 and/or motion sensor 92 are also mounted on the belt and indicate the belt is being worn by a user. In this way, the actual usage of the lift training system is logged together with a log of incorrect lifting angle.

As with the previously discussed lift training system system the microprocessor is programmed with lifting parameters via the compliance computer that when exceeded trigger indicator means 27 to alert the user. The compliance computer is used to interrogate the electronic memory for evaluating and tabulating the results of the lift monitoring session.

It should be noted that the output signals of usage sensors 90 and 92 do not have to be aplied to converter 26 if the signals are already in digital form. In addition the usage sensors can be used on the belt disclosed in Figures 1-3.

## Claims

1. A physical movement monitoring system for monitoring the lifting movement of a user characterised in that said system comprises a belt means (10) adapted and constructed to be secured about a user's waist, at least one electromyographic sensor (22) which produces a rectified and time averaged signal forming a muscle force signal, the sensor being provided with at least one electrode (12, 14, 16) which is secured to the belt in such a manner that when the belt is worn the electrode is located adjacent to a user's lower back so that the sensor senses muscles force exerted by the lower back during a lifting operation.

2. A physical movement monitoring system as defined by claim 1 characterised in that a goniometer means (20) is provided for measuring the lifting angle of a user's lumbar axis, the goniometer means (20) being secured to the belt (10) in such a manner that when the belt is worn the goniometer means (20) is located adjacent to user's side so that the output of the goniometer means corresponds to the lifting angle of a user's lumbar axis during a lifting motion.

3. A physical movement monitoring system as defined by claim 1 or claim 2 characterised in that the electromyographic sensor (22) is provided with three electrodes (12, 14, 16) all of which are secured to the belt in such a manner that when the belt is worn the electrodes are located adjacent to a user's back for sensing muscle force exerted by a user's lower back during a lifting operation.

4. A physical movement monitoring system as defined by claim 3 characterised in that a control means (24) is provided for receiving the muscle force signal from the electromyographic sensor (22) and the output signal from the goniometer means (20) and evaluating the lifting motion of a user to determine if the lifting motion exceeds predetermined standards.

5. A physical movement monitoring system as defined by claim 4 characterised in that an indicator means (27) is provided which is coupled to the control means (24) and if the lifting motion of a user exceeds predetermined standards, the indicator means signals a user as to this condition.

6. A physical movement monitoring system as defined by claim 5 characterised in that a recording means (25) is provided for recording the muscle force signals from the electromyographic sensor (22) and the output signal of the goniometer means (20) and the time of these signals for later playback.

7. An exercise training system for alerting a user when an exercise is to begin and recording the intensity of the exercise, characterised in that the training system comprises an electromyographic sensor (22) which produces a rectified and time averaged signal forming a muscle force signal, the sensor being provided with at least one electrode (12, 14, 16) that is positioned adjacent to a user's muscle group for indicating muscle force of that muscle group, and a control means (24) having a clock for measuring time intervals and an alerting means (52) for alerting a user that an exercise period has started as determined by the clock for a predetermined time interval loaded into the control means, whereby a user in response to the alterting means contracts and relaxes a predetermined muscle group and the muscle force used in the exercise is sensed by the electromyographic sensor.

8. An exercise training system as defined by claim 7 characterised in that a recording means (25) is provided for recording the muscle force signals indicating muscle force from the electromyographic sensor (22) and time of the muscle force signals.

9. An exercise training system as defined by claim 11 characterised in that a display means (50) is provided for displaying the muscle force exerted by a user when contracting a muscle group adjacent to the electrode of the electromyographic sensor (22).

10. A physical movement monitoring system for monitoring the lifting movement of a user characterised in that said system comprises a belt means (10) adapted and constructed to be secured about a user's waist, a goniometer (20) for measuring horizontal angles being mounted on the belt for measuring the lifting angle of a user's back, and generating an output signal related thereto.

11. A physical movement monitoring system as defined by claim 24 characterised in that a control means (24) is provdied for receiving the output signal from the goniometer (20) and evaluating the lifting motion of a user to determine if the lifting motion exceeds predetermined standards.

12. A physical movement monitoring system as defined by claim 1 characterised in that an indicator means (27) is provided which is coupled to the control means (24) and if the lifting motion of a user exceeds predetermined standards, the indicator means signals a user as to this condition.

*FIG. I.*

*FIG. 2.*

*FIG. 3.*

FIG.4.

EP 0 336 030 A1

FIG.5A.

FIG.5B.

FIG.5C.

FIG.5D.

MONITOR
42

38

PRINTER
46

COMPLIANCE
COMPUTER

44
KEYBOARD

36

INTERFACE

TRAINING
MODULE

EMS
SENSING
ELECTRODES

FIG.6.

60

50

54 — CONTRACT
56 — RELAX

27

72 — START
STOP

34

73
FAULT

FIG.7.

FIG. 8.

EP 0 336 030 A1

START SESSION, READ NV. RAM,
INITIALIZE RELEVANT VARIABLES

GET 1ST EMG VALUE AND SET
TOP AND BOTTOM OF SCALE
TO THIS VALUE

GET NEW EMG VALUE

IS
NEW EMG
< BOTTOM
VALUE ?

N          Y

DISPLAY BOTTOMOST
BAR GRAPH ELEMENT,
SET BOTTOM OF SCALE
TO NEW VALUE

IS
NEW EMG
> TOP
VALUE ?

N          Y

DISPLAY TOPMOST
BAR GRAPH ELEMENT,
SET TOP OF SCALE
TO NEW VALUE

CALCULATE NEW DISPLAY ELEMENT, Y, & DISPLAY

$$Y = \left( \frac{NEW\ VALUE - BOTTOM\ VALUE}{TOP\ VALUE - BOTTOM\ VALUE} \right) * N$$

WHERE N = # BAR GRAPH DISPLAY ELEMENTS

WAIT APPOX. 1/10 SECOND

FIG. 9.

CAST MATERIAL
75

MODULE
VIEWING AREA

LCD
DISPLAY

40

74
GAUZE/COTTON
WRAP

12

ELECTRODE
STRIP

14

16

GAUZE/COTTON WRAP

CAST MATERIAL

FIG. 10.

82

80

12  14  16

FIG. 11.

82

81

12  14  16

FIG. 12.

USAGE SENSORS

90 — TEMP. SENSOR

MOTION SENSOR — 92

HORIZONTAL ANGLE 20

ANALOG TO DIGITAL CONVERSION — 26

MEMORY — 25

MICRO PROCESSOR

AUDITORY AND/OR VIBRATIONAL FEEDBACK ELEMENT — 27

COMPLIANCE COMPUTER — 38

36

24

FIG.13.

EP 0 336 030 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 074 407 (KONNO)<br>* Figures 1-5; page 6, line 21 - page 12, line 16 * | 1 | A 61 B 5/10 |
| A | | 3,4,7-9 | |
| X | US-A-4 655 227 (GRACOVETSKY)<br>* Figures 1-5; column 5, line 5 - column 6, line 64; column 7, line 62 - column 9, line 25; column 10, line 44 - column 16, line 12 * | 1 | |
| A | | 2-4,6,7,10-12 | |
| X | US-A-4 702 108 (AMUNDSEN et al.)<br>* Abstract; figure 34; column 14, line 45 - column 17, line 12 * | 7 | |
| A | | 1,3-9 | |
| X | DE-A-3 442 549 (KRÄMER)<br>* Figures 1-4; page 3, line 23 - page 5, line 7; page 5, line 21 - page 7, line 5 * | 10 | TECHNICAL FIELDS SEARCHED (Int. Cl.4) |
| A | | 2,4-6,11,12 | A 61 B<br>A 63 B |
| A | US-A-4 170 225 (CRIGLAR)<br>* Figures 1-5; column 2, line 26 - column 3, line 4; column 5, line 32 - column 6, line 53; column 8, lines 1-30 * | 1,3-5,7,9 | |

The present search report has been drawn up for all claims

| Place of search | Date of completion of the search | Examiner |
|---|---|---|
| THE HAGUE | 06-12-1988 | CHEN A.H. |